# DEMANDE DE BREVET EUROPEEN

(11) **EP 1 795 172 A1**
(43) Date de publication de la demande: **13.06.2007**
(21) Numéro de dépôt: 06124926.4
(22) Date de dépôt: 28.11.2006
(51) Int. Cl.: A61K 8/02, A61Q 1/14, A61Q 19/10

(54) **Article soluble de gommage de la peau**

(30) Priorité: 07.12.2005 FR 0553753
(71) Demandeur: L'Oreal, 75008 Paris (FR)
(72) Inventeur: Aubrun-Sonneville, Odile, 92160 Antony (FR); Bordeaux, Dominique, 91450 Soisy sur Seine (FR)
(74) Mandataire: Rasson, Catherine

(57) **Abrégé**

La présente invention porte sur un article de gommage de la peau, comportant :
- un support sous forme d'au moins une nappe comprenant des fibres solubles dans l'eau à une température inférieure ou égale à 30°C, et
- une composition portée par le support, contenant au moins un agent de gommage sous forme de particules.

L'article selon l'invention peut être utilisé notamment pour le nettoyage et le démaquillage de la peau, pour le gommage de la peau et comme peelings.

## Description

La présente invention concerne des articles de gommage de la peau, comprenant un support soluble dans l'eau et des particules gommantes portées par le support, et leurs utilisations notamment dans le domaine cosmétique pour exfolier la peau en douceur.

Dans le domaine cosmétique, il est connu d'utiliser des produits de gommage pour nettoyer la peau en profondeur par un effet plus ou moins abrasif apporté par des particules microniques ou millimétriques. Ces particules sont généralement dures et de forme irrégulière (pierre ponce, polyéthylène, noyaux de fruits broyés, sable, etc...). De ce fait, d'une part, les produits de gommage les contenant ne peuvent pas toujours être utilisés sur des peaux fragiles, réactives ou fines car elles provoquent rougeurs, inconfort, sensations de brûlures, tiraillements ; d'autre part, il est difficile de maintenir les particules en suspension, car celles-ci sédimentent rapidement. Il est possible de résoudre ce problème, en ajoutant des polymères qui vont gélifier la composition et éviter de ce fait la sédimentation des particules, mais le choix de ces polymères est restreint car il faut disposer de polymères ou association de polymères qui donnent un seuil élevé en rhéologie pour assurer une bonne suspension des particules ; il peut s'agir par exemple de la gomme de xanthane en association avec les polymères carboxyvinyliques tels que les carbomers. Toutefois, les compositions gélifiées par de tels polymères ne sont pas toujours efficaces à pH acide ou en présence d'électrolytes, car les acides ou les électrolytes peuvent déstabiliser notamment les polymères ioniques comme les carbomers qui sont sensibles à de tels composés. Par ailleurs, les textures possibles sont peu diversifiées en raison du choix restreint de polymères.

Il subsiste donc le besoin de disposer de produits de gommage, n'ayant pas les inconvénients de ceux de l'art antérieur, et notamment de disposer de produits de gommage qui permettent une exfoliation de la peau en douceur.

La présente demande répond à ce besoin. En effet, la demanderesse a trouvé de manière surprenante qu'il était possible de préparer des produits de gommage doux en associant au moins un support contenant des fibres solubles dans l'eau et un agent de gommage sous forme de particules, et d'obtenir ainsi par dissolution de ces articles dans l'eau ou dans un milieu aqueux, une composition cosmétique ayant de bonnes propriétés gommantes. Ces articles sont en outre particulièrement doux quand on utilise comme particules, des particules molles telles que celles en polymère super-absorbant.

L'utilisation de ce type d'articles permet de lever les contraintes de formulation liées à la suspension de particules. Les articles obtenus peuvent également adaptés aux consommateurs à peau fragile grâce à la douceur des particules utilisées quand on utilise des particules molles. Enfin, ces articles permettent d'élargir la palette de textures disponibles pour le gommage car il ne se produit plus d'incompatibilté des composés.

Ainsi, l'invention réside, selon l'un de ses aspects, dans un article de gommage de la peau, comportant :
- un support sous forme d'au moins une nappe comprenant des fibres solubles dans l'eau à une température inférieure ou égale à 30°C, et
- une composition portée par le support, contenant au moins un agent de gommage sous forme de particules.

Le ou les agents de gommage peuvent être incorporés sur le support tels quels sur le support mais ils peuvent aussi être incorporés en mélange avec d'autres composés. La composition portée par le support peut donc ne comprendre que le ou les agents de gommage qui constituent à eux seuls la composition, ou bien elle peut contenir le ou les agents de gommage en mélange avec d'autres composés. Cette composition est généralement anhydre, en entendant ici par « anhydre » une composition qui contient une quantité d'eau inférieure ou égale à 5 %, allant donc de 0 à 5 % du poids de la composition. Cette composition et le ou les agents de gommage peuvent se présenter avantageusement sous forme de poudre ou de granulés ou éventuellement sous forme pâteuse. Cette composition constitue notamment une composition cosmétique ou dermatologique.

On entend dans la présente demande par « portée par le support », le fait que la composition peut être soit mise sur le support soit introduite dans la cavité formée par le support quand celui-ci comporte au moins deux nappes de fibres.

On entend par « température inférieure ou égale à 30°C », une température ne dépassant pas 30°C mais n'étant pas inférieure à 0°C, par exemple allant de plus de 0°C à 30°C, mieux de 5°C à 30°C et encore mieux de 10°C à 30°C ou 10°C à 20 °C.

Cet article constitue un produit de gommage soluble ou partiellement soluble. En outre, il présente l'avantage de pouvoir constituer une composition de gommage exempte de conservateur.

Comme indiqué ci-après, selon un mode préféré de réalisation de l'invention, l'article comprend de préférence comme agent de gommage, au moins un polymère superabsorbant. On entend par « polymère superabsorbant », un polymère réticulé qui, une fois hydraté, forme des billes molles ayant un diamètre moyen en nombre de 1 µm à 5 mm.

Les termes « gommage » et « exfoliation » doivent être considérés comme synonymes dans la présente demande, de même que les termes « gommant » et « exfoliant ».

Par ailleurs, les termes « nappe » et « couche » doivent être considérés comme synonymes dans la présente demande. Le support de la présente invention se présente sous forme d'une ou plusieurs nappes de fibres, ce qui est différent des films fins hydrosolubles qui ne sont pas sous forme de nappes de fibres. Par rapport à ces films fins hydrosolubles, les supports à base de nappes de fibres hydrosolubles selon l'invention présentent l'avantage de permettre l'incorporation de constituants incompatibles, d'être plus simples à mettre oeuvre car ils ne nécessitent pas de prémélange ni de mise en solution des composants, ni de chauffage pour évaporer le solvant, le procédé étant également plus rapide et moins coûteux. En outre, les supports selon l'invention ont l'avantage de permettre une plus grande diversité dans le choix de la forme et de l'aspect de l'article car la nappe de fibres peut avoir une épaisseur et une densité variables donnant accès à une grande variété de forme et de taille, alors que le film fin est difficile à sécher si l'épaisseur est trop grande, et il est fragile et difficile à manipuler si la taille est trop grande.

Selon un mode préféré de réalisation de l'invention, l'article se présente sous la forme d'au moins deux nappes définissant entre elles une cavité, l'une au moins des nappes comprenant des fibres solubles dans l'eau à une température inférieure ou égale à 30°C,
- la cavité contenant une composition contenant au moins un agent de gommage.

Les nappes sont assemblées à leur périphérie et forment ainsi une cavité permettant d'introduire la composition contenant l'agent de gommage.

Les nappes peuvent être formées entièrement de fibres solubles dans l'eau ou bien l'une des nappes peut être constituée entièrement de fibres solubles et l'autre nappe peut être constituée de fibres insolubles ou à la fois de fibres solubles et de fibres insolubles dans l'eau, ou bien les deux nappes peuvent être constituées à la fois de fibres solubles et de fibres insolubles.

Selon un mode préféré de réalisation, au moins une des nappes est constituée exclusivement de fibres solubles dans l'eau.

Par humidification ou dissolution de l'article selon l'invention dans l'eau ou dans une composition aqueuse, on obtient une composition pour application topique, notamment cosmétique ou dermatologique. Cette composition est notamment utile pour application sur la peau.

Ainsi, l'invention a encore pour objet, selon un autre de ses aspects, une composition de gommage obtenue par la dissolution dans l'eau, d'un article tel que défini ci-dessus, c'est-à-dire une composition obtenue par dissolution d'un support sous forme d'au moins une nappe comprenant des fibres solubles dans l'eau à une température inférieure ou égale à 30°C , le dit support portant au moins un agent de gommage sous forme de particules. La composition cosmétique ou dermatologique obtenue par dissolution de l'article peut être obtenue à partir d'un support comprenant une ou plusieurs couches de fibres. La température de dissolution de l'article dans l'eau est généralement la température ambiante (20 à 30°C) mais peut être supérieure à la température ambiante si l'on le souhaite selon l'utilisation envisagée.

Dans un exemple de mise en oeuvre de l'invention, l'article selon l'invention est mis en contact avec l'eau avant son utilisation. Il est ainsi d'abord solubilisé avant d'être appliqué sur la peau du visage ou du corps. Sa solubilisation est généralement entière mais peut être partielle si le support comporte des fibres insolubles.

Dans une autre variante de mise en oeuvre de l'invention, l'article selon l'invention est mis au contact de la peau du visage ou du corps, qui a été préalablement humidifiée.

Ainsi, l'invention a encore pour objet, selon un autre de ses aspects, un procédé cosmétique de gommage de la peau, comportant :
- la formation d'une composition cosmétique par dissolution dans l'eau, d'un article comprenant un support comportant au moins une nappe comprenant des fibres solubles dans l'eau à une température inférieure ou égale à 30°C et au moins un agent de gommage sous forme de particules,
- l'application de la composition ainsi formée sur la peau.

L'invention a aussi pour objet un procédé cosmétique de gommage de la peau, comportant :
- l'humidification de la peau,
- l'application sur la peau humidifiée, d'un article comprenant un support comportant au moins une nappe comprenant des fibres solubles dans l'eau à une température inférieure ou égale à 30°C, et au moins un agent de gommage sous forme de particules.

Par «dissolution dans l'eau à une température inférieure ou égale à 30°C », il faut comprendre une solubilisation dans l'eau à une température allant jusqu'à 30°C avec l'aide d'une agitation manuelle et/ou d'une friction du support le cas échéant, dans un laps de temps typiquement inférieur à 5 mn, de préférence inférieur à 1 mn, de préférence inférieur à 30 secondes. L'invention n'exclut pas qu'une eau de température supérieure à 30 °C soit utilisée pour dissoudre le support.

L'article selon l'invention étant destiné à une application topique, il est physiologiquement acceptable. On entend par « physiologiquement acceptable » un article compatible avec les matières kératiniques telles que la peau, les lèvres, les ongles, le cuir chevelu et/ou les cheveux. Il en est de même du support, ainsi que de la composition et des agents de gommage.

L'article selon l'invention ne contient pas d'adhésif, mais il peut adhérer à la peau quand il est humidifié.

L'article est flexible, c'est-à-dire souple. Par «souple», il faut comprendre un article pouvant être comprimé ou pouvant fléchir sans se rompre, capable de s'adapter aux reliefs du corps humain. Un article souple réalisé sous la forme d'une nappe fibreuse peut dans certains exemples de réalisation être replié sur lui-même au moins une fois sans se casser en deux morceaux.

Cet article est généralement à usage unique.

Après sa fabrication, l'article peut être par exemple conditionné en vrac dans une boîte ou dans un emballage individuel. Le cas échéant, les articles sont conditionnés en chapelet. Les articles peuvent encore être repliés sur eux-mêmes et intercalés, de telle sorte que le retrait d'un article amène le suivant dans une configuration facilitant sa préhension.

Ainsi, l'invention a encore pour objet, selon un autre de ses aspects, un ensemble comportant :
- un emballage,
- au moins un article tel que défini plus haut.

Dans le cas d'une composition colorée, l'article peut être conditionné dans un emballage comportant, le cas échéant, un témoin coloré représentatif de la couleur de la composition obtenue après dissolution de l'article, ceci afin de renseigner le consommateur avant l'achat.

### Support

Le support se présente sous forme d'une nappe comprenant des fibres hydrosolubles, c'est-à-dire des fibres solubles dans l'eau à une température inférieure ou égale à 30°C, de préférence solubles dans l'eau à une température inférieure ou égale à 20°C, c'est-à-dire ayant une température de dissolution dans l'eau allant de plus de 0°C à 30°C, de préférence de plus 0°C à 20°C, et par exemple de 5 °C à 30 °C, et mieux de 5 à 20 °C.

Le support est de préférence sensiblement non rétractable une fois mouillé.

Le support peut avoir toute forme appropriée pour l'utilisation visée, par exemple une forme rectangulaire, ronde ou ovale, et il a de préférence des dimensions permettant sa préhension entre deux doigts au moins. Ainsi, le support peut avoir par exemple une forme ovoïde d'environ 2 à 10 cm de long et d'environ 0,5 à 4 cm de large, ou une forme de disque d'environ 2 à 10 cm de diamètre, ou une forme de carré d'environ 5 à 15 cm de côté, ou une forme de rectangle d'une longueur d'environ 5 à 15 cm, étant entendu qu'il peut avoir toute autre forme et dimension appropriées pour l'utilisation recherchée.

Le support peut former par exemple un coussinet, un masque, un patch, une charlotte, un doigt de gant ou un gant, une nappe à découper, une lingette, un disque, un oval ou un rectangle. En outre, le support peut présenter une forme qui dépend de la région du corps à traiter.

Le support peut présenter une forme aplatie ou une forme non aplatie, présentant par exemple l'aspect d'un bloc formé d'un amas globulaire de fibres hydrosolubles compactées, incorporant une composition contenant des particules gommantes.

Les fibres du support sont généralement enchevêtrées pour former la nappe de fibres. Comme indiqué plus haut, on entend par « nappe comprenant des fibres solubles dans l'eau », une nappe pouvant être entièrement constituée de fibres solubles dans l'eau ou une nappe pouvant comporter à la fois des fibres solubles dans l'eau et des fibres insolubles dans l'eau, les fibres solubles devant être en plus grande quantité que les fibres insolubles. La nappe de fibres doit comporter au moins 60 % en poids de fibres solubles, de préférence au moins 70 % et mieux au moins 80 % en poids par rapport au poids total des fibres. Elle peut ainsi comporter, par exemple, plus de 95 % en poids, voire plus de 99 % en poids et même 100 % en poids de fibres hydrosolubles par rapport au poids total des fibres du support. Ainsi, le support peut être constitué entièrement de nappes de fibres solubles ou il peut être constituée de nappes comportant un mélange de fibres solubles et de fibres insolubles, les fibres insolubles étant selon la définition de la présente invention, des fibres qui ne sont pas solubles dans l'eau à une température inférieure ou égale à 30°C. Le fait d'avoir des fibres insolubles peut permettre de renforcer le pouvoir exfoliant du produit selon l'invention, du fait que les fibres insolubles vont apporter leur contribution au gommage de la peau à côté des particules présentes dans le produit. Par ailleurs, il peut être intéressant d'avori des fibres insolubles pour avoir une plus grande stabilité des supports dans des conditions d'humidité relative élevée.

Ainsi, le support peut être formé de deux nappes constituées de fibres solubles dans l'eau, ou encore d'une nappe constituée de fibres solubles dans l'eau et d'une nappe comprenant à la fois de fibres solubles et de fibres insolubles, ou bien aussi d'une nappe constituée de fibres solubles dans l'eau et d'une nappe constituée de fibres insolubles dans l'eau, ou bien de deux nappes comprenant à la fois de fibres solubles et de fibres insolubles. Il peut y avoir aussi plus de deux nappes.

Selon un mode préféré de réalisation de l'invention, le support est dépourvu de fibres insolubles dans l'eau et il est composé uniquement de fibres solubles de l'eau, de sorte qu'il soit entièrement soluble dans l'eau.

Les fibres solubles peuvent être en tout matériau soluble susceptible d'étre filé en fibres. De préférence, les fibres solubles dans l'eau sont réalisées avec de l'alcool polyvinylique (PVA) selon un procédé qui leur confère la solubilité recherchée, le PVA pouvant avoir plusieurs grades de polymérisation.

Des fibres de PVA solubles dans l'eau à une température inférieure ou égale à 30°C sont commercialisées par la société japonaise KURARAY sous la dénomination commerciale KURALON K-II WN2. Le procédé de fabrication de ces fibres comporte la préparation d'une solution à filer par dissolution d'un polymère à base de PVA soluble dans l'eau, dans un premier solvant organique, le filage de la solution dans un second solvant organique pour obtenir des filaments solidifiés et l'étirage humide des filaments dont on enlève le premier solvant puis que l'on sèche et que l'on soumet à un traitement thermique. La section de ces fibres peut être sensiblement circulaire. Ces fibres ont une résistance à la traction d'au moins 2,7 g/dtex (3 g/d). La demande EP-A-0 636 716 décrit de telles fibres hydrosolubles à base de PVA et leur procédé de fabrication.

L'invention n'est pas limitée à l'emploi de PVA, et on peut utiliser aussi des fibres réalisées dans d'autres matériaux hydrosolubles sous réserve que ces matériaux se dissolvent dans de l'eau ayant la température recherchée, par exemple des fibres de polysaccharides commercialisées sous la dénomination LYSORB par la société LYSAC TECHNOLOGIES, INC ou des fibres à base de polymères polyholosides comme le glucomannane ou l'amidon.

La nappe de fibres peut comporter, le cas échéant, un mélange de différentes fibres solubles dans l'eau à des températures différentes (jusqu'à 30°C).

Les fibres peuvent être composites, et elles peuvent comporter par exemple un coeur et une gaine n'ayant pas la même nature, par exemple formés de différents grades de PVA.

Quand la nappe de fibres contient des fibres insolubles, celles-ci peuvent être en toute matière habituellement utilisée comme fibres insolubles ; ce peut être par exemple des fibres de soie, de coton, de laine, de lin, de cellulose extraites notamment du bois, des légumes ou des algues, de polyamide (Nylon^{®}), d'acide polylactique, de cellulose modifiée (rayonne, viscose, acétate notamment d'acétate de rayonne), de poly-p-phénylène téréphtalamide notamment de Kevlar^{®}, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon^{®}, de collagène insoluble, de polyesters, de polychlorure de vinyle ou de vinylidène, d'alcool polyvinylique, de polyacrylonitrile, de chitosane, de polyuréthane, de polyéthylène téréphtalate, de fibres formées d'un mélange des composés mentionnés ci-dessus, comme des fibres de polyamide/polyester ou de viscose/polester. Les non tissés sont décrits de façon générale dans RIEDEL «Nonwoven Bonding Methods & Materials», Nonwoven World (1987), incorporé ici par référence.

Dans un exemple particulier de réalisation de l'invention, la nappe du support est un non-tissé, comportant des fibres hydrosolubles, seules ou en mélange avec des fibres insolubles comme indiqué plus haut, avec au plus de 40% en poids de fibres insolubles par rapport au poids total des fibres constituant la nappe. De préférence, le non-tissé est constitué de fibres hydrosolubles, c'est-à-dire qu'il ne contient pas de fibres insolubles.

Quand le support ne comporte qu'une seule nappe de fibres, la composition contenant l'agent de gommage peut être déposée sur les deux faces du support ou sur une seule face, l'autre face du support pouvant alors être utilisée par exemple pour la préhension de l'article.

Quand le support selon la présente invention comporte deux nappes, il peut s'agir notamment de deux nappes de non tissé, tous les modes de réalisation décrits ci-dessous pouvant être utilisés, les nappes pouvant contenir ou non des fibres insolubles, et même une des nappes pouvant être constituée uniquement de fibres insolubles, du moment que l'autre nappe contient des fibres solubles.

Selon un mode particulier de réalisation de l'invention, chacune des nappes est un non-tissé constitué de fibres solubles à une température inférieure ou égale à 30°C, c'est-à-dire que les nappes ne comportent que des fibres hydrosolubles.

Selon un autre mode de réalisation, une des nappes est entièrement soluble dans l'eau et est un non-tissé constitué de fibres solubles à une température inférieure ou égale à 30°C, et l'autre nappe est insoluble et est est un non-tissé constitué de fibres insolubles.

Selon encore un autre mode de réalisation, le support comporte deux nappes contenant des fibres solubles ou partiellement solubles avec au plus 40 % de fibres insolubles par rapport au poids total des fibres, et en outre une nappe constituée de fibres insolubles, constituant un substrat insoluble. Ainsi, le support peut comporter au moins une couche d'un substrat insoluble dans l'eau, c'est-à-dire ne comportant que des fibres insolubles. Dans un exemple particulier de ce mode de réalisation, le support comprend une nappe soluble d'un non-tissé constitué de fibres solubles dans l'eau à une température inférieure ou égale à 30°C, et une nappe insoluble d'un non-tissé constitué de fibres insolubles dans l'eau.

Une structure multicouche avec au moins une couche formée d'un substrat insoluble dans l'eau peut par exemple être utile pour réaliser un article comportant un support en forme de doigt de gant. La couche formée de fibres hydrosolubles est située à l'extérieur de l'article, étant destinée à se solubiliser lors de l'utilisation, après avoir été mouillée ou en venant au contact d'une région mouillée du corps.

Pour fabriquer les nappes en non-tissé, quelles soient solubles ou insolubles, toutes les techniques appropriées de constitution d'un non-tissé à partir de fibres peuvent être utilisées. Par exemple, les fibres peuvent être formées par extrusion et déposées sur un convoyeur pour former une nappe de fibres qui est ensuite consolidée par une technique classique de liage de fibres, telle que par exemple l'aiguilletage, le liage à chaud, le calandrage ou le liage par jets d'air chaud (en anglais *air through bonding*), technique dans laquelle la nappe passe dans un tunnel où est insufflé de l'air chaud. Cette dernière technique est avantageusement utilisée lorsque la nappe est constituée de fibres bicomposant, par exemple des fibres comprenant au moins deux grades d'alcool polyvinylique (PVA), dont les points de fusion ou de ramollissement sont différents, ces fibres étant par exemple co-extrudées de manière à ce que la fibre soit constituée d'au moins un premier grade localisé au coeur de la fibre et d'au moins un deuxième grade localisé en périphérie de la fibre, sous la forme d'une gaine. Le liage des fibres peut être plus facile lorsque la gaine présente un point de fusion plus faible que le coeur.

La nappe de fibres peut encore être formée par cardage de fibres découpées à une longueur de 10 à 50 mm, puis dépôt des fibres sur un convoyeur où la nappe peut ensuite être consolidée par une technique de liage telle que décrite ci-dessus.

Lorsque le support comporte plusieurs couches, que celles-ci soient toutes réalisées avec des fibres hydrosolubles ou non, les différentes couches peuvent être assemblées de multiples manières, par exemple par soudage, collage ou couture, et ces couches peuvent constituer le cas échéant une ou plusieurs cavités contenant une ou plusieurs compositions cosmétiques ou dermatologiques ou plusieurs composants d'une même composition cosmétique à mélanger extemporanément. Lors d'un assemblage par couture, un fil lui-même hydrosoluble peut être utilisé, le cas échéant.

Quand le support comporte plusieurs nappes de non tissé, celles-ci peuvent être assemblées notamment par thermosoudage à leur périphérie de manière à constituer un coussinet capable de retenir dans une cavité intérieure, une composition contenant le ou les agents de gommage.

Par ailleurs, le support peut comporter en outre au moins une couche d'un substrat insoluble dans l'eau, c'est-à-dire ne comportant que des fibres insolubles, et dans cet exemple de réalisation,le support comprend une nappe d'un non-tissé constitué de fibres solubles dans l'eau à une température inférieure ou égale à 30°C, et une nappe d'un non-tissé constitué de fibres insolubles dans l'eau.

Selon un autre aspect de l'invention, le support est dépourvu d'adhésif, notamment d'adhésif sensible à la pression.

La densité du support pourra dépendre des applications. Le support peut présenter, par exemple, une densité inférieure ou égale à 0,1 g/cm ou bien supérieure à 0,1 g/ cm³. Selon un mode préféré de réalisation de l'invention, le support a une densité inférieure ou égale à 0,1 g/cm³, mieux allant de 0,01 g/cm³ à 0,1 g/cm³, ce qui permet d'avoir un support très aéré, qui, de ce fait, se dissout dans l'eau plus facilement.

La composition contenant au moins un agent de gommage représente entre 10 et 1000 % en poids par rapport au poids du support, et de préférence entre 10 et 500 % en poids par rapport au poids du support en entendant ici par «poids du support», le poids du support seul, sans le poids de la composition contenant l'agent de gommage. Si la composition ne contient que des agents de gommage, ce sont ces derniers qui peuvent représenter entre 10 et 1000 % en poids par rapport au poids du support, et de préférence entre 10 et 500 % en poids par rapport au poids du support.

### Agents de gommage

L'article selon l'invention contient au moins un agent de gommage sous forme de particules. Ces particules peuvent être dures ou molles.

Comme indiqué ci-dessus, la composition portée par le support peut être constituée d'un ou plusieurs agents de gommage, le terme « constituée » signifiant qu'il n'y a pas d'autre composé., et l'agent de gommage ou les agents de gommage peuvent être directement incorporés sur le support. Selon un autre mode de réalisation, la composition comprend un ou plusieurs agents de gommage en mélange avec d'autres composés, et la composition est préparée par mélange des différents composés avant d'être incorporés sur le support.

Les agents de gommage utilisés dans le produit selon l'invention peuvent être choisis parmi toutes les particules exfoliantes d'origines minérale, végétale ou organique, habituellement utilisées dans le domaine cosmétique, et par exemple parmi les polymères pulvérulents gonflant dans l'eau (poudre ou billes), les particules de polyéthylène (billes ou poudres), les sphères de jojoba, les coques de noyaux de fruits broyées, la pierre ponce, les billes de verre, l'oxyde d'aluminium, et leurs mélanges.

Les polymères pulvérulents gonflant dans l'eau après hydratation peuvent notamment se présenter sous forme de billes ayant un diamètre moyen allant d'environ 1 µm à environ 5000 µm (5 mm), de préférence allant de 10 µm à 5 mm. On peut citer en particulier comme polymères pulvérulents gonflant dans l'eau, les polymères superabsorbants, tels que les particules de polyacrylate de sodium réticulé comme par exemple celles commercialisées sous les dénominations Octacare X100, X110 et RM100 par la société Avecia, celles commercialisées sous les dénominations Flocare GB300 et le Flosorb 500 par la société SNF, les amidons greffés par du polyacrylate de sodium (nom INCl Sodium polyacrylate Starch) tels que ceux commercialisés sous les dénominations Sanfresh ST-100C, ST100MC, IM-300MC par la société Sanyo Chemical Industries, les amidons hydrolysés greffés par le copolymère acryloacrylamide/acrylate de sodium (nom INCl : Starch/acrylamide/sodium acrylate copolymer), comme ceux commercialisées sous les dénominations Water Lock A-240, A-180, B-204, D-223, A-100, C-200, D-223, G-400 par la société Grain Processing, et leurs mélanges.

Les agents de gommage peuvent être choisis également parmi les particules de polyéthylène, les sphères de jojoba comme celles commercialisées sous les dénominations « Florasome » par la société Floratech, les coques de noyaux de fruits broyées, la pierre ponce (nom INCI : pumice) comme celle commercialisée sous la dénomination « Ponce 3/B » par la société Eyraud, les billes de verre, l'oxyde d'aluminium comme celui commercialisé sous la dénomination « DERMAGRAIN 900 » par la société Marketech International.

Selon un mode préféré de réalisation de l'invention, l'article comprend au moins un polymère superabsorbant seul ou en mélange avec au moins un agent de gommage choisi parmi les particules de polyéthylène, les sphères de jojoba les coques de noyaux de fruits broyées, la pierre ponce, les billes de verre, l'oxyde d'aluminium.

Les agents de gommage sont en des quantités variables selon le résultat recherché. Leur quantité dépend aussi de l'agent de gommage utilisé, et elle représente de préférence de 2 à 100 % en poids par rapport au poids de la composition, et mieux de 10 à 100 % en poids par rapport au poids de la composition. Quand un polymère superabsorbant est présent comme agent de gommage, sa quantité va de préférence de 2 à 30 % en poids et mieux de 2 à 25 % en poids par rapport au poids total de la composition portée par le support.

La composition contenant l'agent de gommage est de préférence une composition anhydre pulvérulente ou pâteuse. Ainsi, les compositions utilisables dans l'invention peuvent être par exemple :
- des émulsions lyophilisées ou atomisées, telles que celles décrites dans le document FR-A-2,727,312 ou celles à base d'amidon modifié décrites dans le document EP-A-0 938 892. Ces émulsions sont obtenues par lyophilisation ou atomisation d'une émulsion H/E contenant une phase pulvérulente, conduisant à des laits ou des crèmes par mélange avec l'eau lors de leur utilisation.
- des compositions moussantes sous forme de poudres, contenant des tensioactifs pulvérulents, comme celles à base d'amidon, décrites dans le document EP-A-0 925 777, conduisant à de la mousse par mélange avec l'eau lors de leur utilisation.
- des compositions pulvérulentes exemptes d'huile, contenant par exemple des gélifiants hydrophiles et/ou des tensioactifs, et conduisant à des gels ou des lotions par mélange avec l'eau lors de leur utilisation.
- Des compositions obtenues par simple mélange des constituants, ceux-ci étant de préférence sous forme de poudre.

La composition peut éventuellement contenir une certaine quantité d'eau au moment de son imprégnation sur le support. Toutefois, de manière à éviter sa solubilisation prématurée, l'eau introduite sur le support lors de son imprégnation doit être éliminée par les moyens classiquement utilisés pour la déshydratation des compositions contenant de l'eau, comme par exemple le chauffage. Cependant, la composition peut contenir une certaine quantité d'eau qui est généralement de l'eau liée et qui peut provenir notamment des matières premières hygroscopiques qui contiennent de l'eau, telles que les amidons La quantité d'eau finale dans la composition présente sur l'article est d'au maximum 20 % en poids et de préférence au maximum 10 % en poids par rapport au poids total de la composition.

Lorsque la composition doit être déposée sur le support par l'utilisateur lui-même, la composition et le support peuvent être proposés sous la forme d'un kit. La composition est par exemple livrée en une quantité suffisante pour permettre d'en distribuer une pluralité de doses sur un ensemble de supports destinés à être utilisés successivement.

### Additifs

La composition contenant un ou plusieurs agents de gommage peut contenir, selon l'utilisation finale du produit, d'autres composés que les agents de gommage. Ces additifs peuvent être notamment anhydres ou sous forme solide (poudre). Ils peuvent être notamment choisis parmi ceux généralement utilisés dans les domaines cosmétique et dermatologique, tels que, par exemple, les séquestrants, les parfums, les antioxydants, les actifs, les conservateurs, les matières colorantes (comme les pigments et les colorants hydrophiles), les charges minérales et/ou les charges organiques, les composés lipophiles, les gélifiants hydrosolubles. Bien entendu, l'homme de l'art veillera à choisir ce ou ces éventuels additifs et/ou leurs quantités de manière telle que les propriétés avantageuses attachées intrinsèquement à la composition conforme à l'invention ne soient pas, ou substantiellement pas, altérées par la ou les adjonctions envisagées.

Comme gélifiants hydrosolubles, on peut citer par exemple les dérivés de cellulose tels que la carboxyméthylcellulose, les gommes de xanthane, de scléroglucane, de gellane, de rhamsan, les alginates, la gomme de karaya, les gommes de guar modifiées ou non, et leurs dérivés comme l'hydroxypropylguar, les amidons modifiés et leurs mélanges.

Comme amidons modifiés, on peut citer par exemple
- l'amidon de maïs réticulé et acétylé vendu par la société CERESTAR sous le nom C* Flo 06205,
- l'amidon modifié d'amylopectine/amylose, vendu sous la dénomination commerciale Remyline AP par la société Remy,
- l'amidon de blé modifié et précuit, vendu sous la dénomination commerciale Midsol Krisp par la société Midwest Grain Products,
- l'amidon de blé modifié et raffiné, vendu sous la dénomination commerciale Midsol Adhere par la société Midwest Grain Products,
- la poudre d'amidon de blé modifiée, vendue sous la dénomination commerciale Midsol 35 par la société Midwest Grain Products,
- l'amidon de pomme de terre modifié, vendu sous la dénomination commerciale Perfectagel MPT par la société Avebe,
- et leurs mélanges.

La quantité de gélifiant hydrosoluble dans la composition peut aller par exemple de 0,1 à 10 % en poids, mieux de 0,1 à 5% en poids et encore mieux de 0,1 à 3 % en poids par rapport au poids total de la composition.

Par ailleurs, la composition peut contenir un ou plusieurs actifs. Les actifs peuvent être choisis notamment parmi les agents kératolytiques, les hydratants, les apaisants et les antimicrobiens.

Comme hydratants, on peut citer les polyols tels que la glycérine ; les composés agissant sur la fonction barrière, en vue de maintenir l'hydratation du stratum corneum, ou les composés occlusifs, en particulier les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ; les composés augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ; et leurs mélanges.

Comme agents kératolytiques, on peut citer les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique, et leurs mélanges.

Comme agents apaisants utilisables dans la composition selon l'invention, on peut citer par exemple les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, les extraits de plantes telles que *Paeonia suffruticosa* et / ou *lactiflora, Laminaria saccharina, Boswellia serrata, Centipeda cunnighami, Helianthus annuus, Linum usitatissimum, Cola nitida, Epilobium Angustifolium, Aloe vera, Bacopa monieri,* les sels de l'acide salicylique et en particulier le salicylate de zinc, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier muscat, de cassis, d'ecchium, ou de poisson, des extraits de plancton, la capryloyl glycine, le Seppicalm VG (sodium palmitoylproline et nymphea alba) de Seppic, les tocotrienols, le piperonal, un extrait de clou de girofle, les phytostérols, la cortisone, l'hydrocortisone, l'indométhacine et la beta méthasone.

Comme antimicrobiens, on peut citer par exemple le 2,4,4'-trichloro-2'-hydroxy diphényl éther (ou triclosan), le 3,4,4'-trichlorocarbanilide (ou triclocarban) le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO-A-93/18743, le farnesol, les phytosphingosines et leurs mélanges.

Comme vitamines, on peut utiliser les vitamines ou provitamines hydrosolubles ou liposolubles, comme par exemple les vitamines A (rétinol), C (acide ascorbique), B3 ou PP (niacinamide), B5 (panthénol), B6 ou pyridoxine, E (tocophérol), K1, le bêta-carotène, et les dérivés de ces vitamines et notamment leurs esters, et leurs mélanges.

La composition contenant l'agent de gommage peut contenir aussi une ou plusieurs composés lipophiles, corps gras et notamment huiles, ou actif huileux. La quantité de composé lipophile peut aller par exemple de 0,1 à 50 % en poids par rapport au poids total de la composition portée par le support.

On peut utiliser toute sorte d'huiles et de corps gras bien connus de l'homme du métier, comme par exemple les huiles d'origine végétale (par exemple huiles de jojoba, avocat, sésame, tournesol, maïs, soja, carthame, pépins de raisin), les huiles minérales (par exemple vaseline, isoparaffines éventuellement hydrogénées), les huiles de synthèse (par exemple myristate d'isopropyle, octanoate de cétéaryle, polyisobutylène, palmitate ou myristate d'éthyl-hexyle, alkyl-benzoates), les huiles de silicone volatiles ou non volatiles, et les huiles fluorées ou fluorosiliconées, ainsi que les mélanges de ces huiles.

Comme autres corps gras, on peut citer les alcools gras comme l'alcool stéarylique, l'alcool cétylique et leur mélange (alcool cétéarylique), les acides gras, les gommes, par exemple les gommes de silicone comme le mélange PDMS à groupements alpha oméga hydroxylés / PDMS 5 cst (12/88) vendu sous la dénomination DC 1503 par la société Dow Corning, et les gélifiants lipophiles tels que la bentone.

La composition peut contenir aussi un ou plusieurs tensioactifs moussants, de préférence sous forme pulvérulente, c'est-à-dire sous forme de poudre. Comme tensioactifs moussants, on peut utiliser tous ceux habituellement utilisés dans le domaine cosmétique, ces tensioactifs pouvant être anioniques, non ioniques, cationiques, amphotères ou zwitterioniques.

Les tensioactifs préférés sont ceux en poudre, tels que par exemple le lauryl sulfate de sodium comme le produit commercialisé sous la dénomination Empicol LZ D par la société Allbright & Wilson ou sous la dénomination Tensopol USP97 par la société Tensachem ; la cocamidopropylbetaine comme le produit commercialisé sous la dénomination Tegobetain CK D par la société Degussa ; le lauroyl glutamate de sodium comme le produit commercialisé sous la dénomination Amisoft LS 11 par la société Ajinomoto ; le myristoyl glutamate monosodique comme le produit commercialisé sous la dénomination Acylglutamate MS 11 par la société Ajinomoto ; le mélange de laureth sulfate de sodium et de silice, commercialisé sous la dénomination Texapon KE 2713 par la société Cognis ; le disodium cocamido MEA-sulfosuccinate comme le produit commercialisé sous la dénomination Mackanate CM 100 par la société Mac Intyre ; le methyl cocoyl taurate de sodium, comme le produit commercialisé sous la dénomination Tauranol WSP par la société Finetex ; le decyl d-galactoside uronate de sodium comme le produit commercialisé sous la dénomination Decyl d-galactoside uronate de sodium par la société Ard-Soliance ; le lauroyl methyl beta-alanine (forme acide) commercialisé sous la dénomination LMA-H par la société Mitsui Toatsu ; la n-lauroyl-n-hydroxyethyl-beta-alanine commercialisée sous la dénomination LHEA par la société Mitsui Toatsu ; le cocoyl glycinate de sodium commercialisé sous la dénomination Amilite GCS-11(F) par la société Ajinomoto ; le cocoyl isethionate de sodium comme le produit commercialisé sous la dénomination Jordapon CI P par la société BASF ; le lauryl sulfoacétate de sodium, comme le produit commercialisé sous la dénomination Lathanol LAL poudre par la société Stepan ; le myristate de potassium comme le produit commercialisé sous la dénomination Myristate de potassium (DUB MK) par la société Stearinerie Dubois ; le laurate de potassium comme le produit commercialisé sous la dénomination Laurate de potassium (DUB LK) par la société Stearinerie Dubois, et le laurate de sucrose comme le produit commercialisé sous la dénomination Grilloten LSE 87 par la société Degussa.

L'article selon l'invention trouve son application notamment pour le nettoyage et le gommage de la peau (scrub) y compris comme peelings, et il peut constituer notamment un produit de nettoyage de la peau, un produit exfoliant, un produit démaquillant.

Les exemples qui suivent servent à illustrer l'invention sans toutefois présenter un caractère limitatif. Les quantités indiquées sont en % en poids sauf mention contraire, et elles correspondent, sauf mention contraire, à la quantité de matière première et non à la quantité de matière active. Les noms des composés utilisés sont indiqués en nom INCl, en nom chimique ou en nom commercial.

### EXEMPLES

Le produit utilisé dans les exemples a été réalisé avec un support en fibres Kuralon K-II WN2 à base de PVA. Il a été obtenu en thermosoudant à leur périphérie deux couches de grammage 80 g/m².

Le produit se présentait sous la forme d'un disque de 3 cm de diamètre, comportant une cavité dans laquelle a été introduite la composition. Pour l'exemple 1, la quantité de composition introduite dans la cavité a été de 0,1 gramme; pour les exemples 2 et 4, cette quantité a été de 0,3 gramme, et pour l'exemple 3, cette quantité a été de 0,5 gramme.

| | Exemple 1 selon l'invention | Exemple 2 selon l'invention | Exemple 3 selon l'invention | Exemple 4 selon l'invention |
|---|---|---|---|---|
| Polyacrylate de sodium (1) | 5 | 10 | - | - |
| Sorbitol (hydratant) | - | 50 | - | - |
| Carboxyméthyl cellulose (2) | - | 40 | - | - |
| Myristate de potassium (3) | - | - | - | 90 |
| Pierre ponce | - | | - | 10 |
| Poudre de polyéthylène (4) | 50 | - | 10 | - |
| Amidon modifié (5) | - | - | 22,5 | - |
| Huile de vaseline | - | - | 67,5 | - |
| | | | | |
| UTILISATION | Scrub | Gommage doux pour peaux sèches et sensibles | Crème démaquillante exfoliante | Scrub moussant |

| | | | | |
|---|---|---|---|---|
| (1) Octacare MS100 (Avecia) (2) Blanose 9M31F (Hercules) (3) Myristate de potassium (DUB MK) (Stéarineries Dubois) (4) Microthene MN 727 (Equistar) (5) C* Flo 06205 (Cerestar | | | | |

### Modes opératoires :

- Les exemples 1, 2 et 4 ont été obtenus en mélangeant les poudres, puis en introduisant le mélange dans la cavité du support, qui a été ensuite fermée par soudage.
- L'exemple 3 était une poudre obtenue par atomisation d'une émulsion conformément à ce qui est décrit dans EP-A-0 938 892. Comme pour les autres exemples, la poudre a été introduite dans la cavité du support, qui a été ensuite fermée par soudage.

### Modes d'utilisation :

Pour tous les exemples, l'article est préalablement mouillé avec une quantité d'eau supérieure ou égale à 2 ml avant d'être appliquée sur la zone à traiter.

Pour l'exemple 3, la mousse est de préférence produite dans la main par ajouts successifs d'eau en fonction de la quantité de mousse souhaitée. Elle est ensuite appliquée sur le visage.

Pour les autres exemples, l'article humidifié peut être appliqué directement sur la zone à traiter, humidifiée ou non, ou bien cisaillé dans la main avant d'être appliqué sur la zone à traiter.

Dans tous les cas, la peau est ensuite rincée.

## Revendications

1. Article de gommage de la peau, comportant :
- un support sous forme d'au moins une nappe comprenant des fibres solubles dans l'eau à une température inférieure ou égale à 30°C, et
- une composition portée par le support, contenant au moins un agent de gommage sous forme de particules.

2. Article selon la revendication 1, **caractérisé en ce que** les fibres solubles dans l'eau à une température inférieure ou égale à 30°C sont réalisées avec de l'alcool polyvinylique.

3. Article selon la revendication 1 ou 2, **caractérisé en ce que** la nappe de fibres est un non-tissé.

4. Article selon l'une quelconque des revendications précédentes, **caractérisé en ce que** la nappe de fibres comprend des fibres insolubles dans l'eau.

5. Article selon la revendication précédente, **caractérisé en ce que** la quantité de fibres insolubles dans l'eau est d'au plus 40 % en poids par rapport au poids total des fibres du support.

6. Article selon l'une quelconque des revendications précédentes, **caractérisé en ce que** le support comporte au moins deux nappes contenant chacune des fibres solubles dans l'eau à une température inférieure ou égale à 30°C.

7. Article selon l'une quelconque des revendications précédentes, **caractérisé en ce qu'**il comporte :
- au moins deux nappes définissant entre elles une cavité, l'une au moins des nappes comprenant des fibres solubles dans l'eau à une température inférieure ou égale à 30 °C,
- la cavité contenant la composition contenant au moins un agent de gommage sous forme de particules.

8. Article selon la revendication précédente, **caractérisé en ce que** les deux nappes de fibres sont des non-tissés.

9. Article selon la revendication précédente, **caractérisé en ce que** l'une des nappes est un non-tissé constitué de fibres solubles dans l'eau à une température inférieure ou égale à 30°C, et l'autre nappe est un non-tissé constitué de fibres insolubles dans l'eau.

10. Article selon l'une quelconque des revendications 6 à 9, **caractérisé en ce que** les deux nappes sont assemblées à leur périphérie.

11. Article selon la revendication précédente, **caractérisé en ce que** les nappes sont thermosoudées.

12. Article selon l'une quelconque des revendications 1 à 3, 6 ou 7, **caractérisé en ce que** le support est entièrement soluble dans l'eau.

13. Article selon l'une quelconque des revendications précédentes, **caractérisé en ce que** la quantité d'agent de gommage va de 2 à 100 % en poids par rapport au poids total de la composition.

14. Article selon l'une quelconque des revendications précédentes, **caractérisé en ce que** l'agent de gommage est choisi parmi les polymères gonflant dans l'eau, les particules de polyéthylène, les sphères de jojoba, les coques de noyaux de fruits broyées, la pierre ponce, les billes de verre, l'oxyde d'aluminium, et leurs mélanges.

15. Article selon la revendication précédente, **caractérisé en ce que** l'agent de gommage est un polymère gonflant dans l'eau choisi parmi les polymères superabsorbants.

16. Article selon la revendication précédente, **caractérisé en ce que** le polymère superabsorbant est choisi parmi les particules de polyacrylate de sodium réticulé, les amidons greffés par du polyacrylate de sodium, les amidons hydrolysés greffés par le copolymère acryloacrylamide/acrylate de sodium, et leurs mélanges.

17. Composition cosmétique ou dermatologique obtenue par la dissolution dans l'eau, d'un article d'un article selon l'une quelconque des revendications 1 à 16.

18. Procédé cosmétique de gommage de la peau, comportant :
- la formation d'une composition cosmétique par dissolution dans l'eau, d'un support sous forme d'au moins une nappe comprenant des fibres solubles dans l'eau à une température inférieure ou égale à 30°C et au moins un agent de gommage sous forme de particules,
- l'application de la composition ainsi formée sur la peau.

19. Procédé cosmétique de gommage de la peau, comportant :
- l'humidification de la peau,
- l'application sur la peau humidifiée, d'un article comprenant un support comportant au moins une nappe comprenant des fibres solubles dans l'eau à une température inférieure ou égale à 30°C et au moins un agent de gommage sous forme de particules.

20. Ensemble comportant :
- un emballage,
- au moins un article selon l'une quelconque des revendications 1 à 17.
